# EUROPEAN PATENT APPLICATION

(11) **EP 4 656 214 A1**
(43) Date of publication of application: **03.12.2025**
(21) Application number: 24763683.0
(22) Date of filing: 19.02.2024
(51) Int. Cl.: A61M 1/18, B01D 63/02

(54) **ARTIFICIAL LUNG**

(30) Priority: 28.02.2023 JP 2023029277; 28.02.2023 JP 2023029278
(71) Applicant: Terumo Kabushiki Kaisha, Tokyo 151-0072 (JP)
(72) Inventor: OKA, Tomohiro, Elkton, Maryland 21921 (US); MORI, Takehisa, Ashigarakami-gun, Kanagawa 259-0151 (JP); IWASE, Yoji, Fujinomiya-shi, Shizuoka 418-0015 (JP); HATTORI, Shinya, Fujinomiya-shi, Shizuoka 418-0015 (JP)
(74) Representative: KIPA AB
(86) International application number: PCT/JP2024/005754
(87) International publication number: WO 2024/181202

(57) **Abstract**

Provided is an oxygenator capable of suppressing blood stagnation in the vicinity of a top portion of a housing. An oxygenator (1) includes a housing (10), a core (20), a hollow fiber membrane layer (30), a first blood chamber (40), a second blood chamber (50), a blood inflow port (21), and a blood outflow port (14), and in a side view, a straight line (L1) passing from a proximal end point (14P) of the blood outflow port through a center point (10P) of the housing intersects with a virtual center line (L2) of the housing.

## Description

### Technical Field

The present invention relates to an oxygenator.

### Background Art

Conventionally, an extracorporeal circulation system that assists circulation of blood and respiration of a patient has been widely used in order to temporarily maintain life at the time of open heart surgery of a heart disease or in a rapidly progressing circulation failure or cardiopulmonary arrest state.

The extracorporeal circulation system includes an oxygenator that is incorporated in an extracorporeal circulation circuit including a blood removal path, a blood supply path and the like and exchanges gas with blood, and a pump that is incorporated in the extracorporeal circulation circuit and sends blood to the oxygenator (refer to, for example, Patent Literature 1 below). The extracorporeal circulation system performs gas exchange (gives oxygen to blood and removes carbon dioxide) on the removed blood by the oxygenator, and sends the blood to the blood supply path.

### Citation List

### Patent Literature

Patent Literature 1: JP 2014-504906 A

### Summary of Invention

### Technical Problem

In a case where an extracorporeal circulation system is used for a long period of time, there is a problem that thrombus occurs in a region where a blood flow is slow or a stagnation region in the oxygenator.

In particular, in a case of an oxygenator including a cylindrical housing in which blood flowing in from a blood inflow port radially expands, it is problematic that blood stagnates in the vicinity of a top portion of the housing located on an opposite side of a blood outflow port with respect to a center point of the housing.

The present invention has been made in view of the above problems, and an object thereof is to provide an oxygenator capable of suppressing blood stagnation in the vicinity of a top portion of a housing.

### Solution to Problem

The above object of the present invention is achieved by the following means.
(1) An oxygenator including:
   a cylindrical housing;
   a cylindrical core stored in the housing;
   a hollow fiber membrane layer formed by a bundle of a hollow fiber membrane wound around the core;
   a first blood chamber formed between an outer peripheral surface of the core and an inner peripheral surface of the hollow fiber membrane layer;
   a second blood chamber formed between an outer peripheral surface of the hollow fiber membrane layer and an inner peripheral surface of the housing;
   a blood inflow port that is provided on the core so as to extend in a longitudinal direction of the core and communicates with the first blood chamber; and
   a blood outflow port that is provided on the housing so as to extend in a direction intersecting with a longitudinal direction of the housing and in a tangential direction of the housing and communicates with the second blood chamber, in which, in a side view, a straight line passing from a proximal end point of the blood outflow port through a center point of the housing intersects with a virtual center line of the housing.
(2) The oxygenator according to (1), further including:
   a prime port that is provided on the housing and through which air bubbles can flow out during priming, in which an inner peripheral surface of the prime port includes a recessed portion recessed radially outward, and a continuous portion gently formed continuously to the recessed portion, and
   a straight line passing from the proximal end point of the blood outflow port through the center point of the housing is inclined with respect to the virtual center line of the housing at least to a site exceeding the recessed portion in a circumferential direction.
(3) The oxygenator according to (1) or (2), in which an intersection angle of the straight line passing from the proximal end point of the blood outflow port through the center point of the housing with respect to the virtual center line of the housing is larger than 0 degrees and 16 degrees or shorter.
(4) An oxygenator including:
   a cylindrical housing;
   a cylindrical core stored in the housing;
   a hollow fiber membrane layer formed by a bundle of a hollow fiber membrane wound around the core;
   a first blood chamber formed between an outer peripheral surface of the core and an inner peripheral surface of the hollow fiber membrane layer;
   a second blood chamber formed between an outer peripheral surface of the hollow fiber membrane layer and an inner peripheral surface of the housing;
   a blood inflow port that is provided on the core so as to extend in a longitudinal direction of the core and communicates with the first blood chamber; and
   a blood outflow port that is provided on the housing so as to extend in a direction intersecting with a longitudinal direction of the housing and in a tangential direction of the housing and communicates with the second blood chamber, in which, in a side view, a proximal end point of the blood outflow port is offset from a virtual center line of the housing in the tangential direction of the housing.
(5) An oxygenator including:
   a cylindrical housing;
   a cylindrical core stored in the housing;
   a hollow fiber membrane layer formed by a bundle of a hollow fiber membrane wound around the core;
   a first blood chamber formed between an outer peripheral surface of the core and an inner peripheral surface of the hollow fiber membrane layer;
   a second blood chamber formed between an outer peripheral surface of the hollow fiber membrane layer and an inner peripheral surface of the housing;
   a blood inflow port that is provided on the core so as to extend in a longitudinal direction of the core and communicates with the first blood chamber; and
   a blood outflow port that is provided on the housing so as to extend in a direction intersecting with a longitudinal direction of the housing and in a tangential direction of the housing and communicates with the second blood chamber, in which, in a side view, a projection projecting radially inward from an inner peripheral surface of the housing is provided in a region where a straight line passing from a proximal end point of the blood outflow port through a center point of the housing intersects with the inner peripheral surface of the housing, on a side opposite to the proximal end point.
(6) The oxygenator according to (5), in which the projection is formed in a mountain shape so that an amount projecting outward from the center decreases.
(7) The oxygenator according to (5) or (6), in which a projection amount of a most projecting site of the projection is larger than 0% and smaller than 5% of an inner diameter of the housing.

### Advantageous Effects of Invention

According to the oxygenator in (1) described above, in the side view, a blood branch point at which the blood flows in clockwise and counterclockwise directions and stagnation easily occurs can be separated from the top portion of the housing. Therefore, a flow rate of the blood that flows through the top portion of the housing can be increased, and the stagnation of the blood in the top portion of the housing can be suppressed.

According to the oxygenator of (5) described above, since the projection projecting radially inward from the inner peripheral surface of the housing is provided, the blood collides with the projection at a relatively high rate when radially flowing as a distance to the collision site of the blood becomes short as compared with a configuration in which the projection is not provided. By disposing the projection on a top portion side in the circumferential direction of the housing, a low shear rate region is less likely to occur in the top portion of the housing, so that it is possible to suppress blood stagnation in the top portion of the housing.

### Brief Description of Drawings

Fig. 1 is a longitudinal sectional view of an oxygenator according to a first embodiment of the present invention.
Fig. 2 is a schematic sectional side view of the oxygenator according to the first embodiment.
Fig. 3 is a schematic sectional side view of the oxygenator according to the first embodiment.
Fig. 4 is an enlarged view of a part A in Fig. 2.
Fig. 5 is a schematic sectional side view of an oxygenator according to a comparative example.
Fig. 6 is a diagram for explaining a blood branch point.
Fig. 7 is a diagram illustrating a manufacturing device that manufactures the oxygenator according to the first embodiment.
Fig. 8 is a longitudinal sectional view of an oxygenator according to a second embodiment of the present invention.
Fig. 9 is a schematic sectional side view of the oxygenator according to the second embodiment.
Fig. 10 is a schematic sectional side view of the oxygenator according to the second embodiment.
Fig. 11 is a schematic sectional side view of the oxygenator according to the second embodiment for explaining a blood flow.
Fig. 12 is a schematic sectional side view of an oxygenator according to a comparative example.
Fig. 13 is a diagram illustrating a manufacturing device that manufactures the oxygenator according to the second embodiment.
Fig. 14 is a schematic sectional side view of an oxygenator according to a modified example 1 of the second embodiment.

### Description of Embodiments

### <First Embodiment>

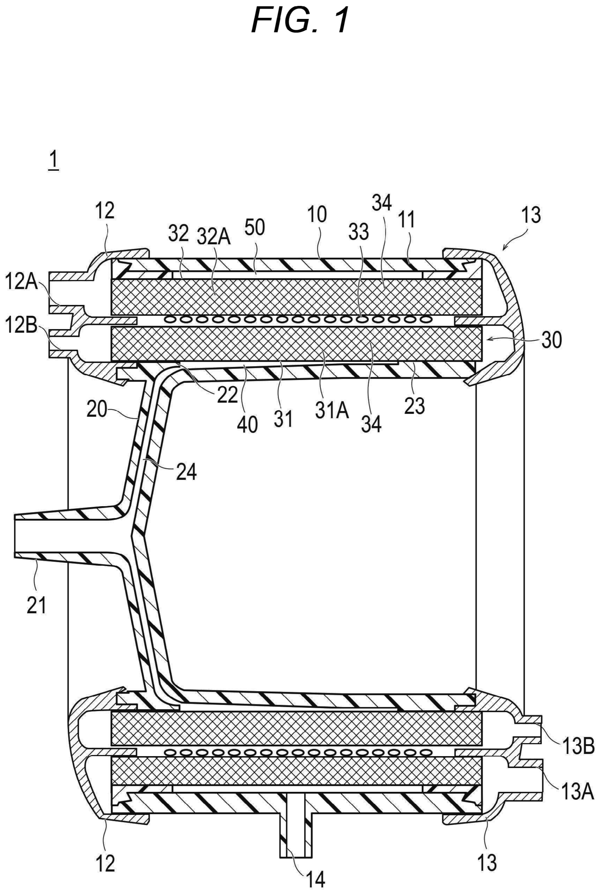

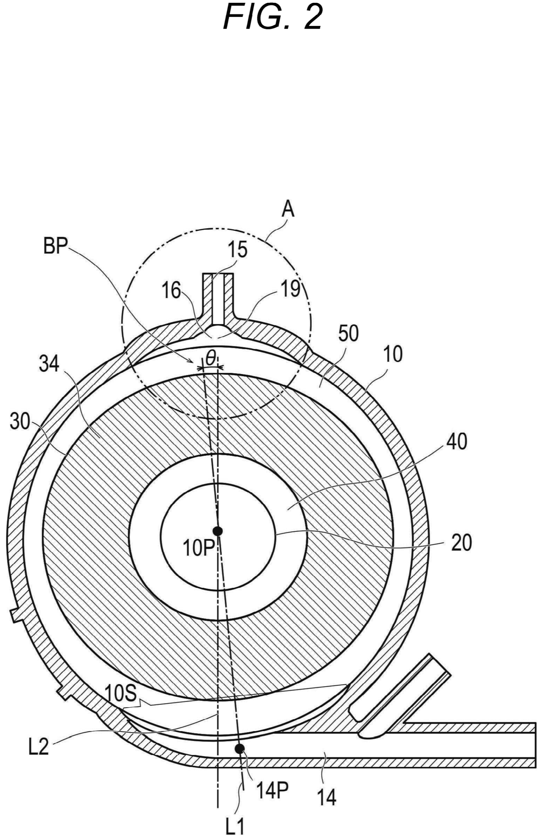

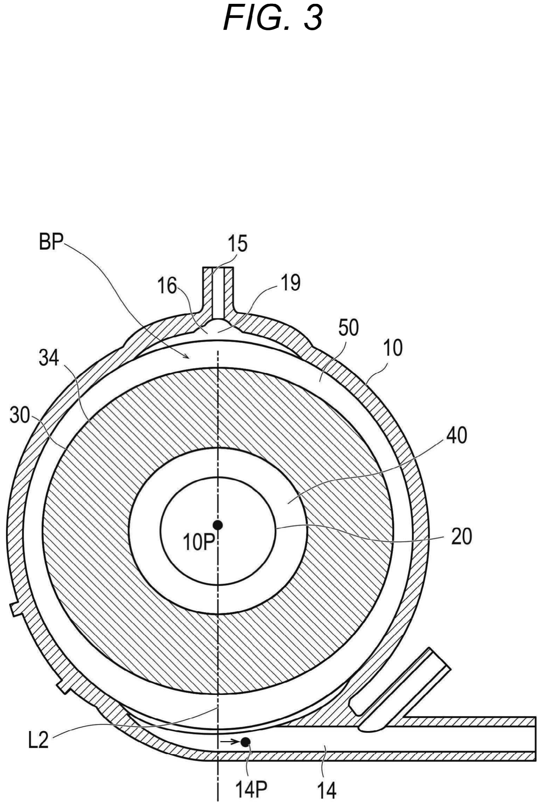

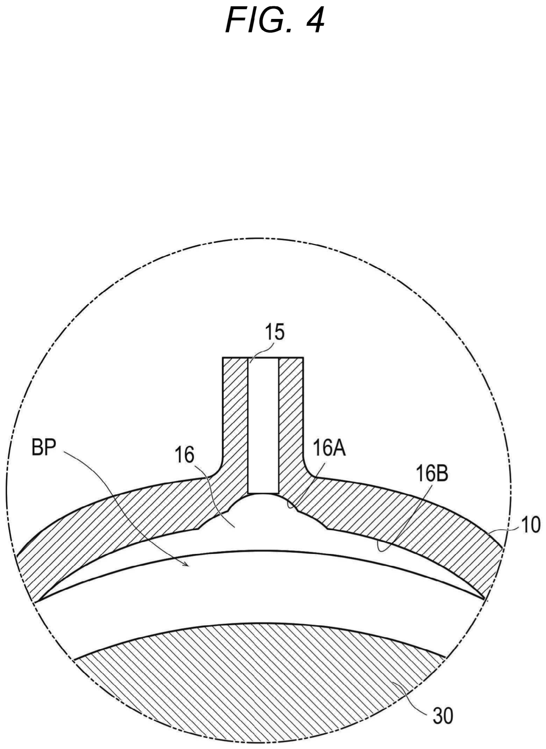

Hereinafter, an oxygenator 1 according to a first embodiment of the present invention will be described with reference to Figs. 1 to 4. Fig. 1 is a longitudinal sectional view of the oxygenator 1 according to the first embodiment of the present invention. Figs. 2 and 3 are schematic sectional side views of the oxygenator 1 according to the first embodiment. Fig. 4 is an enlarged view of a part A in Fig. 2. A dimensional ratio in each drawing is exaggerated for convenience of description, and might be different from an actual ratio.

The oxygenator 1 is incorporated in an extracorporeal circulation circuit and exchanges gas with blood. The oxygenator 1 includes a membrane oxygenator that exchanges gas with the blood by a hollow fiber membrane 34.

As illustrated in Fig. 1, the oxygenator 1 includes a cylindrical housing 10, a core 20 stored in the housing 10, a hollow fiber membrane layer 30 formed by a bundle of the hollow fiber membrane 34 wound around the core 20, a first blood chamber 40 formed between an outer peripheral surface of the core 20 and an inner peripheral surface of the hollow fiber membrane layer 30, and a second blood chamber 50 formed between an outer peripheral surface of the hollow fiber membrane layer 30 and an inner peripheral surface of the housing 10. Note that, the outer peripheral surface of the core 20 and the inner peripheral surface of the hollow fiber membrane layer 30 are partially in contact with each other. In other words, the first blood chamber 40 is formed by a gap in a portion where the outer peripheral surface of the core 20 and the inner peripheral surface of the hollow fiber membrane layer 30 are not in contact with each other. For example, an arterial filter may be interposed between the outer peripheral surface of the hollow fiber membrane layer 30 and the inner peripheral surface of the housing 10.

The housing 10 forms an outer peripheral portion of the oxygenator 1. As illustrated in Fig. 1, the housing 10 includes a cylindrical housing body 11 extending in a longitudinal direction (right-left direction in Fig. 1), and a first header 12 and a second header 13 airtightly connected to both ends of the housing body 11.

As illustrated in Figs. 1 and 2, the housing body 11 includes a blood outflow port 14 provided so as to extend in a direction (up-down direction in Fig. 1) intersecting with (orthogonal to, in Fig. 1) the longitudinal direction of the housing body 11 and in a tangential direction of the housing 10 (right-left direction in Fig. 2), and a prime port 15 from which air bubbles can flow out when performing priming. An arrangement position of the blood outflow port 14 will be described later.

In Fig. 1, a gas inflow port 12A and a heat medium inflow port 12B are provided on an upper side of the first header 12. In Fig. 1, a gas outflow port 13A and a heat medium outflow port 13B are provided on a lower side of the second header 13. The gas outflow port 13A and the heat medium outflow port 13B are located on substantially opposite sides in a circumferential direction with respect to the gas inflow port 12A and the heat medium inflow port 12B.

The gas inflow port 12A, the heat medium inflow port 12B, the gas outflow port 13A, and the heat medium outflow port 13B extend in the longitudinal direction of the housing body 11.

As illustrated in Figs. 2 and 3, the prime port 15 is located on an upper side in the up-down direction in the housing 10.

The prime port 15 is formed in a top portion 19 of the housing 10. The prime port 15 is a hole for allowing air bubbles present in the oxygenator 1 to flow out when performing priming of the inside of the oxygenator 1 with physiologic saline before starting procedure.

On an inner peripheral surface 16 of the prime port 15, as illustrated in Fig. 4, a recessed portion 16A recessed radially outward and a continuous portion 16B gently formed continuously to the recessed portion 16A. According to this configuration, air bubbles present in the vicinity of the continuous portion 16B can also be discharged from the prime port 15 at the time of priming, so that the priming can be performed in a wider range.

The housing 10 is preferably transparent to the extent that a blood flow therein is visible. Note that, the term "transparent" in the present specification includes colorless transparent, colored transparent, and translucent.

A material forming the housing 10 is not particularly limited, and for example, polyolefins such as polyethylene and polypropylene, ester-based resins such as polyethylene terephthalate, styrene-based resins such as polystyrene, MS resin, and MBS resin, polycarbonate and the like can be used.

The core 20 forms a central portion of the oxygenator 1. The core 20 extends in the longitudinal direction of the housing body 11.

The core 20 includes a blood inflow port 21 communicating with the first blood chamber 40 and two support units 22 and 23 that support the hollow fiber membrane layer 30. The blood inflow port 21 extends in the longitudinal direction of the housing body 11. The blood that flows in from the blood inflow port 21 circulates through a circulation unit 24 formed in the core 20 and flows into the first blood chamber 40.

The material forming the core 20 is not particularly limited, and for example, polyolefins such as polyethylene and polypropylene, ester-based resins such as polyethylene terephthalate, styrene-based resins such as polystyrene, MS resin, and MBS resin, polycarbonate and the like can be used.

The housing 10 and the core 20 are attached to each other by the first header 12 and the second header 13.

As illustrated in Fig. 1, the hollow fiber membrane layer 30 is provided between the housing 10 and the core 20. The hollow fiber membrane layer 30 is supported by the support units 22 and 23 provided in the core 20.

As illustrated in Figs. 1 and 2, the hollow fiber membrane layer 30 includes a heat exchange unit 31 disposed on an inner peripheral side and a gas exchange unit 32 disposed on an outer peripheral side. Note that, a spacer 33 may be provided between the heat exchange unit 31 and the gas exchange unit 32.

The heat exchange unit 31 is formed of a bundle of the hollow fiber membrane 34. In the heat exchange unit 31, when the heat medium circulating through a heat medium flow path 31A of the heat exchange unit 31 passes through the heat exchange unit 31, this exchange heat with the blood. The heat exchange unit 31 is formed of a bundle of the hollow fiber membrane 34.

The heat medium that flows in from the heat medium inflow port 12B of the first header 12 is subjected to heat exchange with the blood in the heat exchange unit 31, and then discharged to the outside of the oxygenator 1 through the heat medium outflow port 13B of the second header 13.

The gas exchange unit 32 is formed of a bundle of the hollow fiber membrane 34. In the gas exchange unit 32, oxygen that circulates through a gas flow path 32A of the gas exchange unit 32 is diffused to a blood side when passing through the hollow fiber membrane 34. Carbon dioxide in the blood that circulates through the gas exchange unit 32 is discharged into a lumen of the hollow fiber membrane 34. As a result, in the gas exchange unit 32, gas exchange of oxygen and carbon dioxide is performed with the blood via the hollow fiber membrane 34.

Oxygen that flows in from the gas inflow port 12A of the first header 12 is subjected to gas exchange with carbon dioxide in the blood in the gas exchange unit 32, and carbon dioxide subjected to the gas exchange is discharged to the outside of the oxygenator 1 through the gas outflow port 13A of the second header 13.

The hollow fiber membrane layer 30 is formed by stacking the hollow fiber membrane 34 many times. The hollow fiber membrane 34 is formed by forming a large number of hollow fibers having a gas exchange function into a tubular shape.

A forming material of the hollow fiber membrane 34 is not particularly limited as long as the gas exchange with the blood can be performed, and for example, a hydrophobic polymer material such as polypropylene, polyethylene, polysulfone, polyacrylonitrile, polytetrafluoroethylene, or polymethylpentene can be used.

Hereinafter, an arrangement position of the blood outflow port 14 will be described with reference to Figs. 2 to 4. In the oxygenator 1 according to the first embodiment, as illustrated in Fig. 2, a straight line L1 passing from a proximal end point 14P of the blood outflow port 14 through a center point 10P of the housing 10 intersects with a virtual center line L2 of the housing 10 in a side view. In the present specification, the "virtual center line L2 of the housing 10" refers to a straight line drawn in the up-down direction from the center point 10P of the housing 10. In the present specification, the "proximal end point 14P of the blood outflow port 14" refers to, in an opening 10S of the housing 10 communicating with the blood outflow port 14, on an ideal inner peripheral surface of the housing 10 passing through both ends of the opening 10S, a center point of the both ends.

An angle θ (corresponding to an intersection angle) at which the straight line L1 passing from the proximal end point 14P of the blood outflow port 14 through the center point 10P of the housing 10 intersects with the virtual center line L2 of the housing 10 is not particularly limited, but is preferably larger than 0 degrees and 16 degrees or shorter.

Note that, a lower limit value of the angle θ at which the straight line L1 passing from the proximal end point 14P of the blood outflow port 14 through the center point 10P of the housing 10 intersects with the virtual center line L2 of the housing 10 is preferably set to exceed at least the recessed portion 16A in the circumferential direction. Here, for example, in a case where the angle θ is shifted to the extent not exceeding the recessed portion 16A, a blood branch point BP to be described later coincides with the recessed portion 16A in which the blood stagnates, so that the blood easily stagnates. In contrast, by shifting the angle θ to exceed the recessed portion 16A, the blood branch point BP can be set at a site exceeding the recessed portion 16A, so that a flow rate of the blood in the recessed portion 16A can be increased. Therefore, it is possible to suppress blood stagnation in the recessed portion 16A. Note that, a configuration in which the angle θ is shifted to the extent not exceeding the recessed portion 16A is also included in the present invention.

For example, in a case where the angle θ exceeds 16 degrees, an amount by which the proximal end point 14P of the blood outflow port 14 moves (offset) to the right in Figs. 2 and 3 increases, and there is a possibility that the blood does not suitably flow out from the proximal end point 14P of the blood outflow port 14. Note that, a configuration in which the angle θ exceeds 16 degrees is also included in the present invention.

In the side view of the oxygenator 1 according to the first embodiment described above, the configuration in which the straight line L1 passing from the proximal end point 14P of the blood outflow port 14 through the center point 10P of the housing 10 intersects with the virtual center line L2 of the housing 10 can be rephrased as below with reference to Fig. 3.

That is, in the oxygenator 1 according to the first embodiment, in the side view as illustrated in Fig. 3, the proximal end point 14P of the blood outflow port 14 is offset from the virtual center line L2 of the housing 10 in the tangential direction of the housing (right side in Fig. 3) (refer to an arrow in Fig. 3).

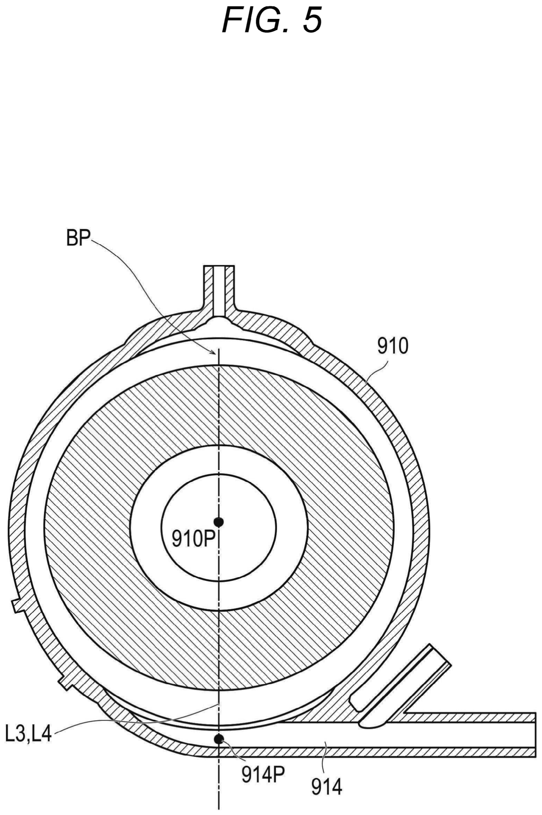

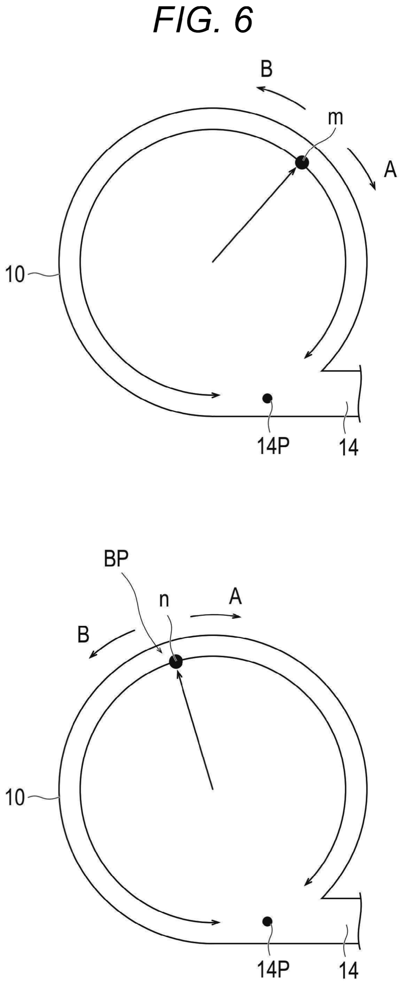

Next, the blood branch point BP, a configuration of an oxygenator according to a comparative example, and an effect of the oxygenator 1 according to the first embodiment will be described with reference to Figs. 5 and 6.

First, the blood branch point BP will be described with reference to Fig. 6. For example, as illustrated in an upper diagram of Fig. 6, the blood that flows to a point m of the second blood chamber 50 easily flows in a direction A after reaching the vicinity of an inner wall of the housing 10. This is because a route to the proximal end point 14P of the blood outflow port 14 in the direction A is shorter than a route to the proximal end point 14P of the blood outflow port 14 in a direction B. In contrast, as illustrated in a lower diagram of Fig. 6, the blood that flows to a point n of the second blood chamber 50 flows in the direction A and the direction B because the route to the proximal end point 14P of the blood outflow port 14 in the direction A and the route to the proximal end point 14P of the blood outflow port 14 in the direction B have the same length, and this point n serves as the blood branch point BP. At the blood branch point BP, since a rate of the flow in a portion interposed between the flow in the direction A and the flow in the direction B is substantially zero, the blood easily stagnates as a result.

Here, for example, as illustrated in Fig. 5, in a case where a straight line L3 passing from a proximal end point 914P of a blood outflow port 914 through a center point 910P of a housing 910 and a virtual center line L4 of the housing 910 are the same straight line, in other words, in a case where the proximal end point 914P of the blood outflow port 914 is not offset from the virtual center line L4 of the housing 910 in the tangential direction of the housing 910, the blood branch point BP is at the same site as the prime port 15. Therefore, since the top portion 19 of the housing 10 in which the blood easily stagnates coincides with the blood branch point BP, the blood more easily stagnates in the top portion 19 of the housing 10.

In contrast, in the oxygenator 1 according to the first embodiment, as illustrated in Fig. 2, in the side view, the straight line L1 passing from the proximal end point 14P of the blood outflow port 14 through the center point 10P of the housing 10 intersects with the virtual center line L2 of the housing 10, in other words, as illustrated in Fig. 3, in the side view, the proximal end point 14P of the blood outflow port 14 is offset from the virtual center line L2 of the housing 10 in the tangential direction of the housing (right side in Fig. 3), so that the blood branch point BP is shifted in a counterclockwise direction from the top portion 19 of the housing 10 as illustrated in Figs. 2 and 3. Therefore, as compared with the oxygenator according to the comparative example, the flow rate of the blood flowing through the top portion 19 of the housing 10 can be increased, and the stagnation of the blood in the top portion 19 of the housing 10 can be suppressed.

Next, the flow of the blood in the oxygenator 1 will be described.

The blood that flows in from the blood inflow port 21 circulates through the circulation unit 24 of the core 20 and is guided to the first blood chamber 40. When the blood guided to the first blood chamber 40 moves through the gap between the hollow fiber membrane 34 in the order of the heat exchange unit 31 and the gas exchange unit 32 radially outward in the hollow fiber membrane layer 30, heat exchange with the heat medium is performed in the heat exchange unit 31, and gas exchange with oxygen is performed in the gas exchange unit 32.

In contrast, the heat medium that flows in from the heat medium inflow port 12B of the first header 12 is subjected to heat exchange with the blood in the heat exchange unit 31, and then discharged to the outside of the oxygenator 1 through the heat medium outflow port 13B of the second header 13.

Oxygen that flows in from the gas inflow port 12A of the first header 12 is subjected to gas exchange with carbon dioxide in the blood in the gas exchange unit 32, and carbon dioxide subjected to the gas exchange is discharged to the outside of the oxygenator 1 through the gas outflow port 13A of the second header 13.

After reaching the second blood chamber 50, the blood subjected to the gas exchange flows out of the oxygenator 1 from the blood outflow port 14 communicating with the second blood chamber 50 and returns to a human body.

### <Manufacturing Device and Manufacturing Method of Oxygenator 1>

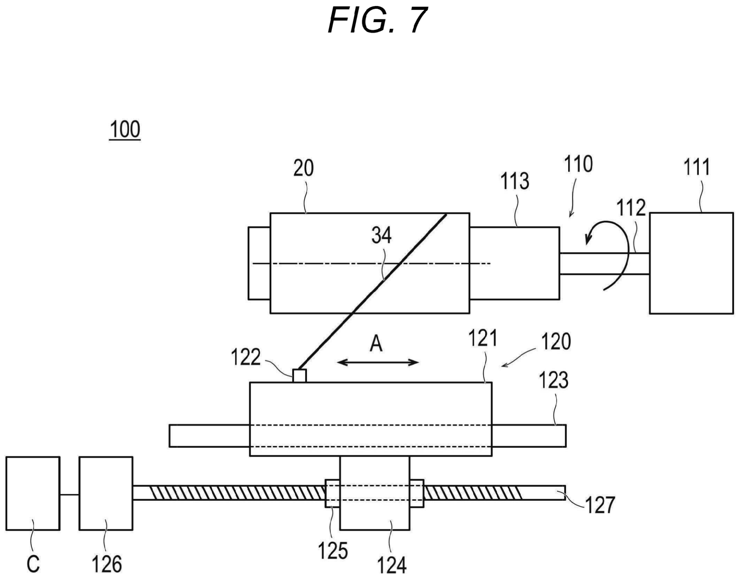

Next, a manufacturing device 100 and a manufacturing method of the oxygenator 1 will be described with reference to Fig. 7. Fig. 7 is a diagram illustrating the manufacturing device 100 that manufactures the oxygenator 1 according to the first embodiment.

First, a configuration of the manufacturing device 100 of the oxygenator will be described with reference to Fig. 7.

As illustrated in Fig. 7, the manufacturing device 100 includes a rotating device 110 that rotates the core 20, and a winding device 120 that winds the hollow fiber membrane 34 around the core 20.

As illustrated in Fig. 7, the rotating device 110 includes a motor 111, a motor shaft 112 that transmits rotation of the motor 111, and a connection member 113 connected to the motor shaft 112, the member to which the core 20 is attached. The core 20 is rotated about an axis by the motor 111 in a state of being attached to the connection member 113.

The winding device 120 includes a main body 121 including the hollow fiber membrane 34 therein, and a discharge unit 122 that discharges the hollow fiber membrane 34. The discharge unit 122 is configured to be movable in the axial direction as indicated by an arrow A in Fig. 7.

The main body 121 is fixed to a linear table 124 and a ball nut 125 that move on a linear rail 123. A motor 126 drives and a ball screw shaft 127 rotates, so that the ball nut 125 is movable in parallel with the axial direction of the main body 121. The drive of the motor 126 is adjusted by a controller C. The controller C is, for example, a computer.

The manufacturing device 100 preferably further includes a fixing device that fixes the hollow fiber membrane 34 wound around the core 20 with a fixing yarn, disclosed in WO 2018/062271. Detailed description of the fixing device is omitted.

Next, the manufacturing method of the oxygenator 1 will be described.

First, the core 20 is set on the connection member 113 of the manufacturing device 100.

Next, while the core 20 is rotated by the motor 111, the hollow fiber membrane 34 is discharged from the discharge unit 122 of the winding device 120, and the hollow fiber membrane 34 is spirally wound around the outer periphery of the core 20.

When the winding of the hollow fiber membrane 34 is finished, both ends of the hollow fiber membrane layer 30 are fixed with urethane, and then the both ends are cut. By this step, the end of the hollow fiber membrane layer 30 is exposed, so that the heat medium or oxygen can enter.

Finally, the oxygenator 1 is obtained by attaching the housing 10 to the outer periphery of the core 20 on which the hollow fiber membrane layer 30 is formed.

As described above, the oxygenator 1 according to the first embodiment includes the cylindrical housing 10, the cylindrical core 20 stored in the housing 10, the hollow fiber membrane layer 30 formed by the bundle of the hollow fiber membrane 34 wound around the core 20, the first blood chamber 40 formed between the outer peripheral surface of the core 20 and the inner peripheral surface of the hollow fiber membrane layer 30, the second blood chamber 50 formed between the outer peripheral surface of the hollow fiber membrane layer 30 and the inner peripheral surface of the housing 10, the blood inflow port 21 that is provided on the core 20 so as to extend in the longitudinal direction of the core 20 and communicates with the first blood chamber 40, and the blood outflow port 14 that is provided on the housing 10 so as to extend in the direction intersecting with the longitudinal direction of the housing 10 and in the tangential direction of the housing 10 and communicates with the second blood chamber 50. The straight line L1 passing from the proximal end point 14P of the blood outflow port 14 through the center point 10P of the housing 10 intersects with the virtual center line L2 of the housing 10 in the side view. According to the oxygenator 1 configured as described above, in the side view, the blood branch point BP at which the blood flows in clockwise and counterclockwise directions and stagnation easily occurs can be separated from the top portion 19 of the housing 10. Therefore, the flow rate of the blood that flows through the top portion 19 of the housing 10 can be increased, and the stagnation of the blood in the top portion 19 of the housing 10 can be suppressed.

The oxygenator 1 further includes the prime port 15 provided on the housing 10 through which air bubbles can flow out at the time of the priming. The inner peripheral surface 16 of the prime port 15 includes the recessed portion 16A recessed radially outward and the continuous portion 16B gently formed continuously to the recessed portion 16A. The straight line L1 passing from the proximal end point 14P of the blood outflow port 14 through the center point 10P of the housing 10 inclines with respect to the virtual center line L2 of the housing 10 up to a section exceeding the recessed portion 16A in the circumferential direction. According to the oxygenator 1 configured as described above, the blood branch point BP can be set at a site exceeding the recessed portion 16A and the flow rate of the blood in the recessed portion 16A can be increased.

As described above, the oxygenator 1 according to the first embodiment includes the cylindrical housing 10, the cylindrical core 20 stored in the housing 10, the hollow fiber membrane layer 30 formed by the bundle of the hollow fiber membrane 34 wound around the core 20, the first blood chamber 40 formed between the outer peripheral surface of the core 20 and the inner peripheral surface of the hollow fiber membrane layer 30, the second blood chamber 50 formed between the outer peripheral surface of the hollow fiber membrane layer 30 and the inner peripheral surface of the housing 10, the blood inflow port 21 that is provided on the core 20 so as to extend in the longitudinal direction of the core 20 and communicates with the first blood chamber 40, and the blood outflow port 14 that is provided on the housing 10 so as to extend in the direction intersecting with the longitudinal direction of the housing 10 and in the tangential direction of the housing 10 and communicates with the second blood chamber 50. In the side view, the proximal end point 14P of the blood outflow port 14 is offset from the virtual center line L2 of the housing 10 in the tangential direction of the housing 10. According to the oxygenator 1 configured as described above, in the side view, the blood branch point BP at which the blood flows in clockwise and counterclockwise directions and stagnation easily occurs can be separated from the top portion 19 of the housing 10. Therefore, the flow rate of the blood that flows through the top portion 19 of the housing 10 can be increased, and the stagnation of the blood in the top portion 19 of the housing 10 can be suppressed.

### <Second Embodiment>

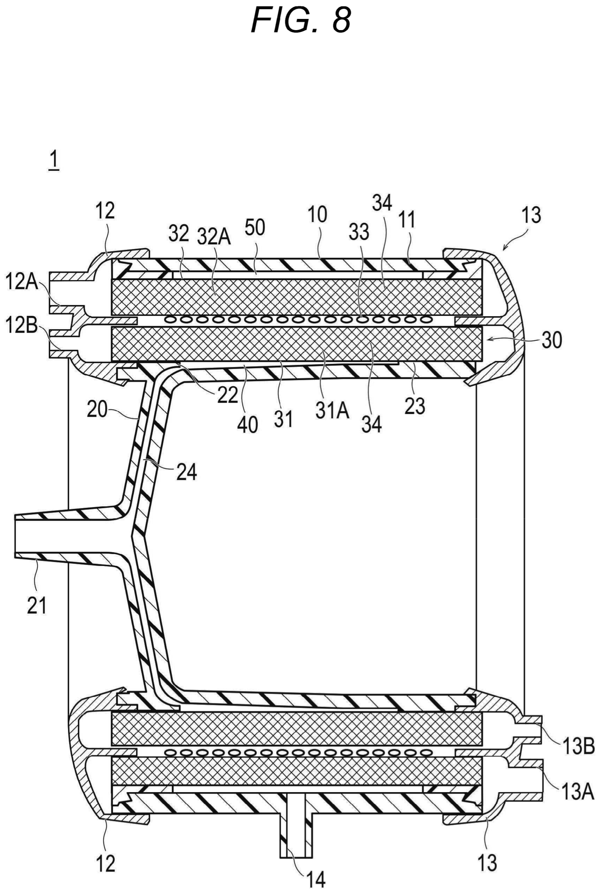

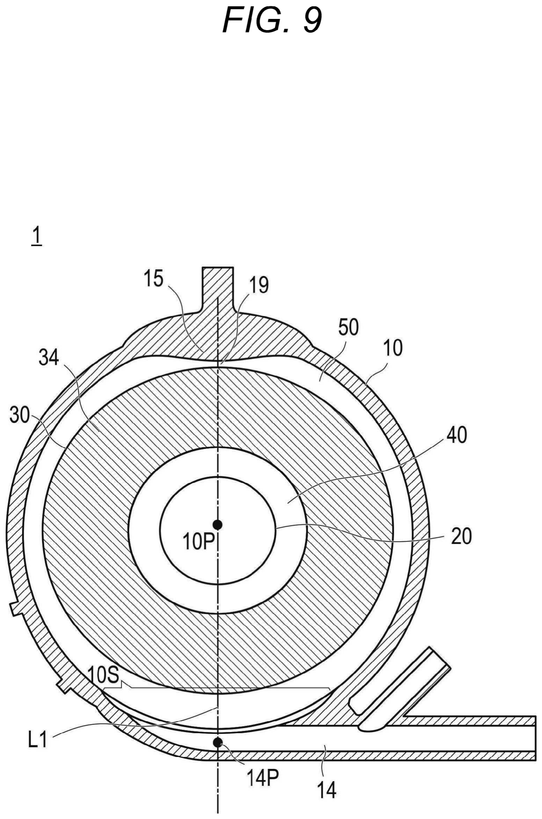

Hereinafter, an oxygenator 1 according to a second embodiment of the present invention will be described with reference to Figs. 8 to 11. Fig. 8 is a longitudinal sectional view of the oxygenator 1 according to the second embodiment of the present invention. Figs. 9 to 11 are schematic sectional side views of the oxygenator 1 according to the second embodiment. A dimensional ratio in each drawing is exaggerated for convenience of description, and might be different from an actual ratio.

The oxygenator 1 is incorporated in an extracorporeal circulation circuit and exchanges gas with blood. The oxygenator 1 includes a membrane oxygenator that exchanges gas with the blood by a hollow fiber membrane 34.

As illustrated in Fig. 8, the oxygenator 1 includes a cylindrical housing 10, a core 20 stored in the housing 10, a hollow fiber membrane layer 30 formed by a bundle of the hollow fiber membrane 34 wound around the core 20, a first blood chamber 40 formed between an outer peripheral surface of the core 20 and an inner peripheral surface of the hollow fiber membrane layer 30, and a second blood chamber 50 formed between an outer peripheral surface of the hollow fiber membrane layer 30 and an inner peripheral surface of the housing 10. Note that, the outer peripheral surface of the core 20 and the inner peripheral surface of the hollow fiber membrane layer 30 are partially in contact with each other. In other words, the first blood chamber 40 is formed by a gap in a portion where the outer peripheral surface of the core 20 and the inner peripheral surface of the hollow fiber membrane layer 30 are not in contact with each other. An arterial filter may be interposed between the outer peripheral surface of the hollow fiber membrane layer 30 and the inner peripheral surface of the housing 10.

The housing 10 forms an outer peripheral portion of the oxygenator 1. As illustrated in Fig. 8, the housing 10 includes a cylindrical housing body 11 extending in a longitudinal direction (right-left direction in Fig. 8), and a first header 12 and a second header 13 airtightly connected to both ends of the housing body 11.

As illustrated in Figs. 8 and 9, the housing body 11 includes a blood outflow port 14 provided so as to extend in a direction (up-down direction in Fig. 8) intersecting with (orthogonal to in Fig. 8) the longitudinal direction of the housing body 11 and in a tangential direction of the housing 10 (light-right direction in Fig. 9), and a projection 15 projecting from the inner peripheral surface of the housing 10 radially inward in a side view.

In Fig. 8, a gas inflow port 12A and a heat medium inflow port 12B are provided on an upper side of the first header 12. In Fig. 8, a gas outflow port 13A and a heat medium outflow port 13B are provided on a lower side of the second header 13. The gas outflow port 13A and the heat medium outflow port 13B are located on substantially opposite sides in a circumferential direction with respect to the gas inflow port 12A and the heat medium inflow port 12B.

The gas inflow port 12A, the heat medium inflow port 12B, the gas outflow port 13A, and the heat medium outflow port 13B extend in the longitudinal direction of the housing body 11.

As illustrated in Fig. 9, the projection 15 is located on an opposite side of a proximal end point 14P of the blood outflow port 14 with respect to a center point 10P of the housing 10. Herein, the proximal end point 14P of the blood outflow port 14 refers to, in an opening 10S of the housing 10 communicating with the blood outflow port 14, on an ideal inner peripheral surface of the housing 10 passing through both ends of the opening 10S, a center point of the both ends.

In other words, as illustrated in Fig. 9, the projection 15 is provided in a region where a straight line L1 passing from the proximal end point 14P of the blood outflow port 14 through the center point 10P of the housing 10 intersects with the inner peripheral surface of the housing 10.

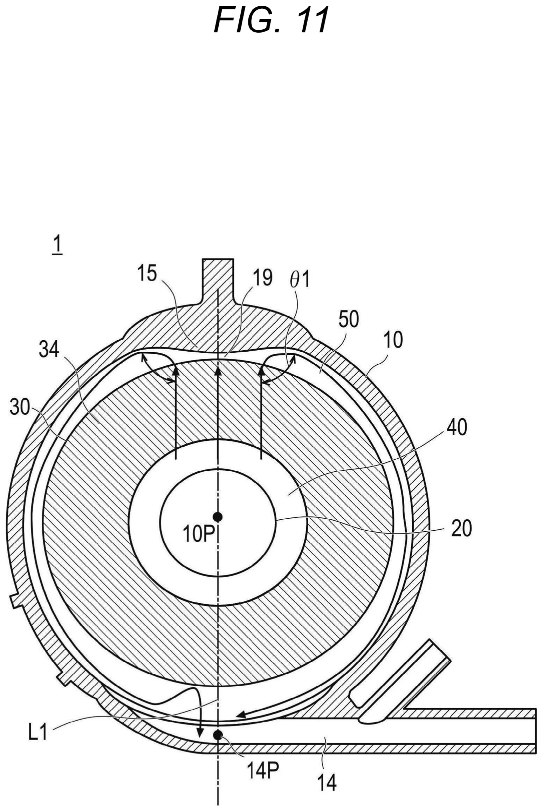

As illustrated in Fig. 9, the projection 15 is preferably formed in a mountain shape so that an amount projecting outward in the circumferential direction (right-left direction in Fig. 9) from the center decreases. Since the projection 15 is formed in a mountain shape in this manner, the flow of the blood when the radially flowing blood collides with the projection 15 and branches to the left and right forms an obtuse angle (indicated by θ1 in Fig. 11) larger than 90°, so that a vortex is hardly generated, and stagnation of the blood in a top portion 19 of the housing 10 can be suppressed. Note that, the projection 15 may have, as a three-dimensional shape, a mountain shape, or a convex surface curved in the longitudinal direction of the housing 10. The same applies to a modified example described below.

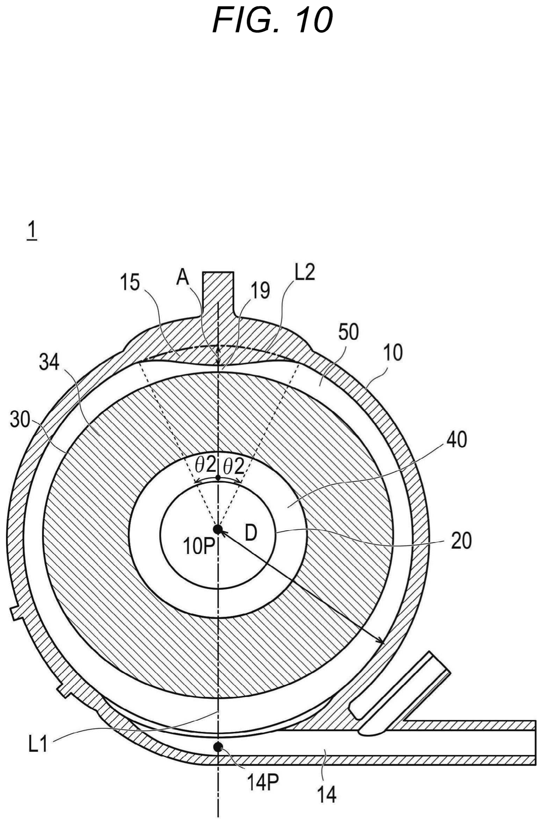

As illustrated in Fig. 10, a projection amount A of a most projecting site of the projection 15 (the center of the projection 15 in a width direction) is preferably larger than 0% and smaller than 5% of an inner diameter D of the housing 10. Here, as illustrated in Fig. 10, the projection amount A indicates a distance to a site most projecting from a virtual line L2 when the inner periphery of the housing 10 where the projection 15 is not provided is extended to a site where the projection 15 is provided as the virtual line L2. Here, in a case where the projection amount A is larger than 5% of the inner diameter D of the housing 10, the projection 15 might come into contact with the hollow fiber membrane layer 30 disposed on the inner periphery of the housing 10, which is not preferable. Note that, a configuration in which the projection amount A is larger than 5% of the inner diameter D of the housing 10 is also included in the present invention.

As illustrated in Fig. 10, in the side view, the projection 15 is provided in a range of -60° to 60° (an angle θ2 in Fig. 10) around the center point 10P of the housing 10 with respect to the straight line L1 passing from the proximal end point 14P of the blood outflow port 14 through the center point 10P of the housing 10. Note that, the region where the projection 15 is provided is not limited to the above range.

In the second embodiment, the projection 15 is formed integrally with the housing 10 on the inner peripheral surface of the housing 10. Note that, the projection 15 may be formed separately from the housing 10 and fixed to the inner peripheral surface of the housing 10 with an adhesive or the like.

The housing 10 is preferably transparent to the extent that a blood flow therein is visible. Note that, the term "transparent" in the present specification includes colorless transparent, colored transparent, and translucent.

A material forming the housing 10 is not particularly limited, and for example, polyolefins such as polyethylene and polypropylene, ester-based resins such as polyethylene terephthalate, styrene-based resins such as polystyrene, MS resin, and MBS resin, polycarbonate and the like can be used.

The core 20 forms a central portion of the oxygenator 1. The core 20 extends in the longitudinal direction of the housing body 11.

The core 20 includes a blood inflow port 21 communicating with the first blood chamber 40 and two support units 22 and 23 that support the hollow fiber membrane layer 30. The blood inflow port 21 extends in the longitudinal direction of the housing body 11. The blood that flows in from the blood inflow port 21 circulates through a circulation unit 24 formed on the core 20 and is guided to the first blood chamber 40.

The material forming the core 20 is not particularly limited, and for example, polyolefins such as polyethylene and polypropylene, ester-based resins such as polyethylene terephthalate, styrene-based resins such as polystyrene, MS resin, and MBS resin, polycarbonate and the like can be used.

The housing 10 and the core 20 are attached to each other by the first header 12 and the second header 13.

As illustrated in Fig. 8, the hollow fiber membrane layer 30 is provided between the housing 10 and the core 20. The hollow fiber membrane layer 30 is supported by the support units 22 and 23 provided in the core 20.

As illustrated in Figs. 8 and 9, the hollow fiber membrane layer 30 includes a heat exchange unit 31 disposed on an inner peripheral side and a gas exchange unit 32 disposed on an outer peripheral side. Note that, a spacer 33 may be provided between the heat exchange unit 31 and the gas exchange unit 32.

The heat exchange unit 31 is formed of a bundle of the hollow fiber membrane 34. In the heat exchange unit 31, when the heat medium circulating through a heat medium flow path 31A of the heat exchange unit 31 passes through the heat exchange unit 31, this exchange heat with the blood. The heat exchange unit 31 is formed of a bundle of the hollow fiber membrane 34.

The heat medium that flows in from the heat medium inflow port 12B of the first header 12 is subjected to heat exchange with the blood in the heat exchange unit 31, and then discharged to the outside of the oxygenator 1 through the heat medium outflow port 13B of the second header 13.

The gas exchange unit 32 is formed of a bundle of the hollow fiber membrane 34. In the gas exchange unit 32, oxygen that circulates through a gas flow path 32A of the gas exchange unit 32 is diffused to a blood side when passing through the hollow fiber membrane 34. Carbon dioxide in the blood that circulates through the gas exchange unit 32 is discharged into a lumen of the hollow fiber membrane 34. As a result, in the gas exchange unit 32, gas exchange of oxygen and carbon dioxide is performed with the blood via the hollow fiber membrane 34.

Oxygen that flows in from the gas inflow port 12A of the first header 12 is subjected to gas exchange with carbon dioxide in the blood in the gas exchange unit 32, and carbon dioxide subjected to the gas exchange is discharged to the outside of the oxygenator 1 through the gas outflow port 13A of the second header 13.

The hollow fiber membrane layer 30 is formed by stacking the hollow fiber membrane 34 many times. The hollow fiber membrane 34 is formed by forming a large number of hollow fibers having a gas exchange function into a tubular shape.

A forming material of the hollow fiber membrane 34 is not particularly limited as long as the gas exchange with the blood can be performed, and for example, a hydrophobic polymer material such as polypropylene, polyethylene, polysulfone, polyacrylonitrile, polytetrafluoroethylene, or polymethylpentene can be used.

Next, the flow of the blood in the oxygenator 1 will be described.

The blood that flows in from the blood inflow port 21 circulates through the circulation unit 24 of the core 20 and is guided to the first blood chamber 40. When the blood guided to the first blood chamber 40 moves through the gap between the hollow fiber membrane 34 in the order of the heat exchange unit 31 and the gas exchange unit 32 radially outward in the hollow fiber membrane layer 30, heat exchange with the heat medium is performed in the heat exchange unit 31, and gas exchange with oxygen is performed in the gas exchange unit 32.

In contrast, the heat medium that flows in from the heat medium inflow port 12B of the first header 12 is subjected to heat exchange with the blood in the heat exchange unit 31, and then discharged to the outside of the oxygenator 1 through the heat medium outflow port 13B of the second header 13.

Oxygen that flows in from the gas inflow port 12A of the first header 12 is subjected to gas exchange with carbon dioxide in the blood in the gas exchange unit 32, and carbon dioxide subjected to the gas exchange is discharged to the outside of the oxygenator 1 through the gas outflow port 13A of the second header 13.

After reaching the second blood chamber 50, the blood subjected to the gas exchange flows out of the oxygenator 1 from the blood outflow port 14 communicating with the second blood chamber 50 and returns to a human body.

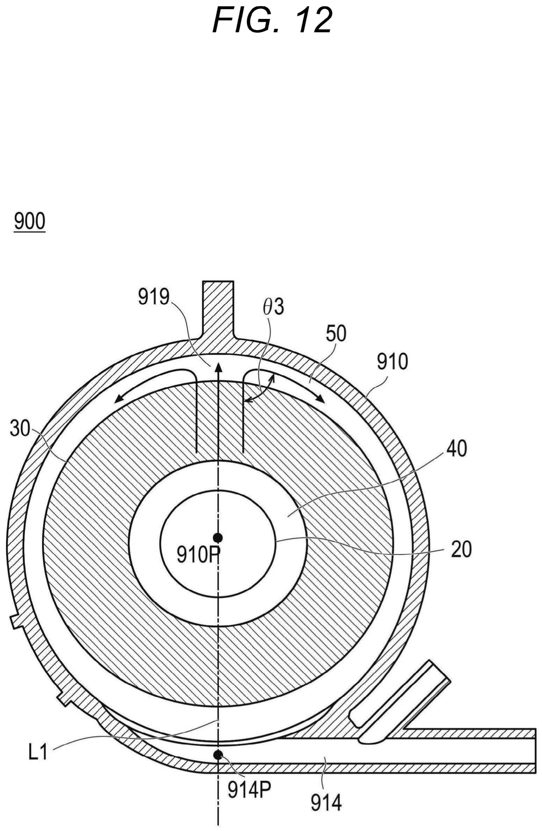

Hereinafter, an effect of the oxygenator 1 according to the second embodiment is described while describing a configuration of an oxygenator according to a comparative example with reference to Figs. 11 and 12. Fig. 11 is a diagram illustrating a flow of the blood in the oxygenator 1 according to the second embodiment, and Fig. 12 is schematic sectional side view of the oxygenator according to the comparative example.

As described above, the oxygenator 1 according to the second embodiment includes the projection 15 projecting radially inward from the inner peripheral surface of the housing 10 as illustrated in Figs. 9 and 10.

Here, for example, as illustrated in Fig. 12, in a case of an oxygenator 900 without projection included, blood stagnates in the vicinity of a top portion 919 of a housing 910 located on an opposite side of a proximal end point 914P of a blood outflow port 914 with respect to a center point 910P of the housing 910. This is because, when the blood radially flows, the flow rate gradually decreases due to a long time until collision with an inner peripheral surface of the housing 910, the flow rate in the vicinity of the site where the blood branches becomes substantially zero, and a shear rate is low.

In contrast, since the oxygenator 1 according to the second embodiment includes the projection 15 projecting radially inward from the inner peripheral surface of the housing 10, a time until collision with the inner peripheral surface of the housing 10 (in other words, the projection 15) is shortened, the decrease in flow rate is suppressed, and the decrease in shear rate can be suppressed as compared with the oxygenator 900 according to the comparative example, so that the stagnation of the blood in the top portion 19 of the housing 10 can be suppressed.

For example, as illustrated in Fig. 12, in a case of the oxygenator 900 including no projection, as illustrated in Fig. 12, the flow of the blood when the radially flowing blood collides with the inner peripheral surface of the housing 910 of the oxygenator 900 and branches to the left and right forms an acute angle (indicated by θ3 in Fig. 12) smaller than 90°, so that a vortex is generated and the blood stagnates.

In contrast, the oxygenator 1 according to the second embodiment includes the projection 15 projecting radially inward from the inner peripheral surface of the housing 10, and the projection 15 has a mountain shape, so that the flow of the blood when the radially flowing blood collides with the projection 15 and branches to the left and right forms an obtuse angle (indicated by θ1 in Fig. 11) larger than 90°, a vortex is hardly generated, and blood stagnation can be suppressed.

### <Manufacturing Device and Manufacturing Method of Oxygenator 1>

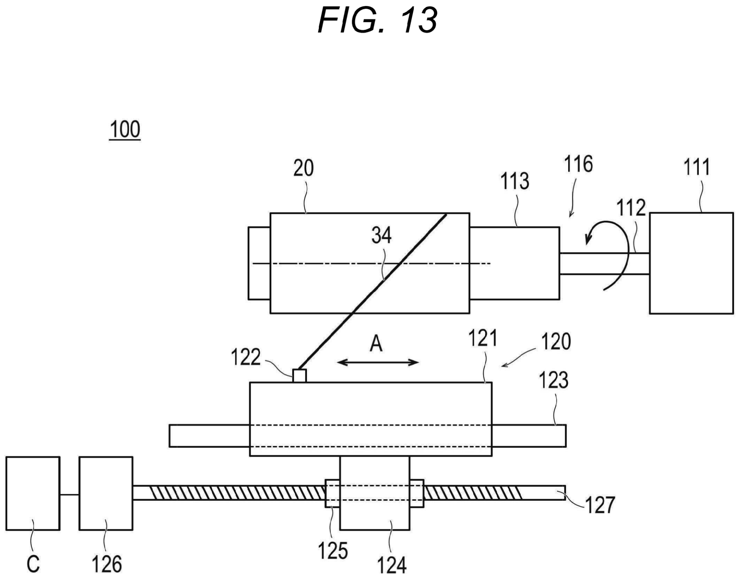

Next, a manufacturing device 100 and a manufacturing method of the oxygenator 1 will be described with reference to Fig. 13. Fig. 13 is a diagram illustrating the manufacturing device 100 that manufactures the oxygenator 1 according to the second embodiment.

First, a configuration of the manufacturing device 100 of the oxygenator will be described with reference to Fig. 13.

As illustrated in Fig. 13, the manufacturing device 100 includes a rotating device 116 that rotates the core 20, and a winding device 120 that winds the hollow fiber membrane 34 around the core 20.

As illustrated in Fig. 13, the rotating device 116 includes a motor 111, a motor shaft 112 that transmits rotation of the motor 111, and a connection member 113 connected to the motor shaft 112, the member to which the core 20 is attached. The core 20 is rotated about an axis by the motor 111 in a state of being attached to the connection member 113.

The winding device 120 includes a main body 121 including the hollow fiber membrane 34 therein, and a discharge unit 122 that discharges the hollow fiber membrane 34. The discharge unit 122 is configured to be movable in the axial direction as indicated by an arrow A in Fig. 13.

The main body 121 is fixed to a linear table 124 and a ball nut 125 that move on a linear rail 123. A motor 126 drives and a ball screw shaft 127 rotates, so that the ball nut 125 is movable in parallel with the axial direction of the main body 121. The drive of the motor 126 is adjusted by a controller C. The controller C is, for example, a computer.

The manufacturing device 100 preferably further includes a fixing device that fixes the hollow fiber membrane 34 wound around the core 20 with a fixing yarn, disclosed in WO 2018/062271. Detailed description of the fixing device is omitted.

Next, the manufacturing method of the oxygenator 1 will be described.

First, the core 20 is set on the connection member 113 of the manufacturing device 100.

Next, while the core 20 is rotated by the motor 111, the hollow fiber membrane 34 is discharged from the discharge unit 122 of the winding device 120, and the hollow fiber membrane 34 is spirally wound around the outer periphery of the core 20.

When the winding of the hollow fiber membrane 34 is finished, both ends of the hollow fiber membrane layer 30 are fixed with urethane, and then the both ends are cut. By this step, the end of the hollow fiber membrane layer 30 is exposed, so that the heat medium or oxygen can enter.

Finally, the oxygenator 1 is obtained by attaching the housing 10 to the outer periphery of the core 20 on which the hollow fiber membrane layer 30 is formed.

As described above, the oxygenator 1 according to the second embodiment includes the cylindrical housing 10, the cylindrical core 20 stored in the housing 10, the hollow fiber membrane layer 30 formed by the bundle of the hollow fiber membrane 34 wound around the core 20, the first blood chamber 40 formed between the outer peripheral surface of the core 20 and the inner peripheral surface of the hollow fiber membrane layer 30, the second blood chamber 50 formed between the outer peripheral surface of the hollow fiber membrane layer 30 and the inner peripheral surface of the housing 10, the blood inflow port 21 that is provided on the core 20 so as to extend in the longitudinal direction of the core 20 and communicates with the first blood chamber 40, and the blood outflow port 14 that is provided on the housing 10 so as to extend in the direction intersecting with the longitudinal direction of the housing 10 and in the tangential direction of the housing 10 and communicates with the second blood chamber 50. In the side view, the projection 15 projecting radially inward from the inner peripheral surface of the housing 10 is provided on the opposite side of the proximal end point 14P in the region where the straight line L1 passing from the proximal end point 14P of the blood outflow port 14 through the center point 10P of the housing 10 intersects with the inner peripheral surface of the housing 10. According to the oxygenator 1 configured as described above, since the projection 15 projecting radially inward from the inner peripheral surface of the housing 10 is provided, so that the distance to the collision site of the blood becomes short, and the blood collides with the projection at a relatively higher rate when the blood flows radially. By disposing the projection 15 on the top portion 19 side in the circumferential direction of the housing 10, a low shear rate region is less likely to occur in the top portion 19 of the housing 10, so that it is possible to suppress blood stagnation in the top portion 19 of the housing 10.

The projection 15 is formed in a mountain shape so that the amount projecting outward from the center decreases. According to the oxygenator 1 configured as described above, the flow of the blood when the radially flowing blood collides with the projection 15 and branches to the left and right forms an obtuse angle (indicated by θ1 in Fig. 11) larger than 90°, so that a vortex is hardly generated, and stagnation of the blood can be suppressed.

### <Oxygenator 2 according to Modified Example 1>

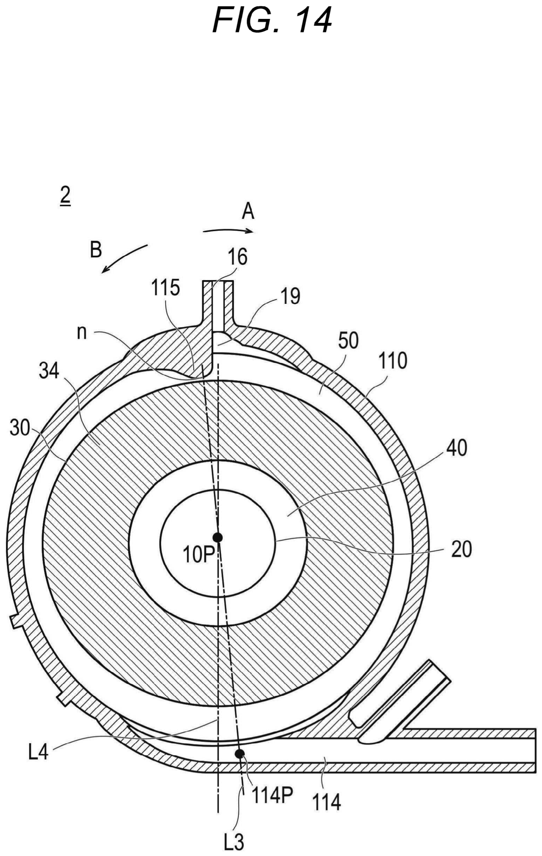

Next, a configuration of an oxygenator 2 according to a modified example 1 of the second embodiment will be described with reference to Fig. 14. Fig. 14 is a schematic sectional side view of the oxygenator 2 according to the modified example 1 of the second embodiment.

The oxygenator 2 according to the modified example 1 is different from the oxygenator 1 according to the above-described embodiment in the configuration of the housing 110 and the like. Note that, the same components as those of the oxygenator 1 according to the above-described embodiment are denoted by the same reference numerals, and the description thereof will be omitted.

As illustrated in Fig. 14, the oxygenator 2 includes a cylindrical housing 110, a core 20 stored in the housing 110, a hollow fiber membrane layer 30 formed by a bundle of the hollow fiber membrane 34 wound around the core 20, a first blood chamber 40 formed between an outer peripheral surface of the core 20 and an inner peripheral surface of the hollow fiber membrane layer 30, and a second blood chamber 50 formed between an outer peripheral surface of the hollow fiber membrane layer 30 and an inner peripheral surface of the housing 110. Since the configurations of the core 20, the hollow fiber membrane layer 30, the first blood chamber 40, and the second blood chamber 50 are the same as those of the oxygenator 1 according to the embodiment, the description thereof will be omitted.

The housing 110 includes a blood outflow port 114, a projection 115 projecting radially inward from the inner peripheral surface of the housing 110, and a prime port 16 through which air bubbles can flow out when performing priming.

In the oxygenator 2 according to the modified example 1, as illustrated in Fig. 14, a straight line L3 passing from a proximal end point 114P of the blood outflow port 114 through a center point 110P of the housing 110 intersects with a virtual center line L4 of the housing 110 in a side view. In the present specification, the "virtual center line L4 of the housing 110" refers to a straight line drawn in the up-down direction from the center point 110P of the housing 110.

As illustrated in Fig. 14, the projection 115 is provided on a left side with respect to the virtual center line L4 of the housing 110.

As illustrated in Fig. 14, the prime port 16 is located on an upper side in the up-down direction in the housing 110.

The prime port 16 is formed in a top portion 19 of the housing 110. The prime port 16 is a hole for allowing air bubbles present in the oxygenator 1 to flow out when performing priming of the inside of the oxygenator 1 with physiologic saline before starting procedure.

In this configuration, in the side view, a point at which the straight line L3 passing from the proximal end point 114P of the blood outflow port 114 through the center point 110P of the housing 110 intersects with the projection 115 is defined as a point n. The blood that flows to the point n of the second blood chamber 50 flows in a direction A and a direction B because a route to the proximal end point 114P of the blood outflow port 114 in the direction A and a route to the proximal end point 114P of the blood outflow port 114 in the direction B have the same length, and this point n serves as the blood branch point.

At the blood branch point, since a rate of the flow in a portion interposed between the flow in the direction A and the flow in the direction B is substantially zero, the blood easily stagnates as a result.

According to the oxygenator 2 configured as described above, the blood branch point is shifted in a counterclockwise direction from the top portion 19 of the housing 110 as illustrated in Fig. 14. Therefore, the flow rate of the blood flowing through the top portion 19 of the housing 110 can be increased, and the stagnation of the blood in the top portion 19 of the housing 110 can be suppressed.

According to the oxygenator 2 according to the modified example 1, by providing the projection 115, it is possible to suppress blood stagnation in the top portion 19 of the housing 110 as in the above-described embodiment. Furthermore, in the side view, since the straight line L3 passing from the proximal end point 114P of the blood outflow port 114 through the center point 110P of the housing 110 intersects with the virtual center line L4 of the housing 110, the blood branch point BP is shifted in the counterclockwise direction from the top portion 19 of the housing 110 as illustrated in Fig. 14, the flow rate of the blood flowing through the top portion 19 of the housing 110 can be increased, and the stagnation of the blood in the top portion 19 of the housing 110 can be suppressed.

The oxygenator 1 according to the present invention has been described above through the embodiment; however, the present invention is not limited to only the configurations described in the embodiment, and can be changed as appropriate on the basis of the recitation in claims.

For example, in the second embodiment described above, the projection 15 is formed in a mountain shape, but the projection is not particularly limited as long as the projection projects radially inward from the inner peripheral surface of the housing 10.

The present application is based on Japanese Patent Application No. 2023-29277 and Japanese Patent Application No. 2023-29278 filed on February 28, 2023, the disclosure content of which is incorporated herein by reference in its entirety.

### Reference Signs List

- 1, 2: Oxygenator
- 10, 110: Housing
- 10P, 110P: Center point
- 14, 114: Blood outflow port
- 14P, 114P: Proximal end point
- 15: Prime port
- 15, 115: Projection
- 16: Inner peripheral surface of prime port
- 16A: Recessed portion
- 16B: Continuous portion
- 19: Top portion
- 20: Core
- 21: Blood inflow port
- 30: Hollow fiber membrane layer
- 34: Hollow fiber membrane
- 40: First blood chamber
- 50: Second blood chamber
- BP: Blood branch point
- A: Projection amount of projection
- L1: Straight line passing from proximal end point of blood outflow port through center point of housing
- L2: Virtual center line of housing

## Claims

1. An oxygenator comprising:
a cylindrical housing;
a cylindrical core stored in the housing;
a hollow fiber membrane layer formed by a bundle of a hollow fiber membrane wound around the core;
a first blood chamber formed between an outer peripheral surface of the core and an inner peripheral surface of the hollow fiber membrane layer;
a second blood chamber formed between an outer peripheral surface of the hollow fiber membrane layer and an inner peripheral surface of the housing;
a blood inflow port that is provided on the core so as to extend in a longitudinal direction of the core and communicates with the first blood chamber; and
a blood outflow port that is provided on the housing so as to extend in a direction intersecting with a longitudinal direction of the housing and in a tangential direction of the housing and communicates with the second blood chamber, wherein, in a side view, a straight line passing from a proximal end point of the blood outflow port through a center point of the housing intersects with a virtual center line of the housing.

2. The oxygenator according to claim 1, further comprising:
a prime port that is provided on the housing and through which air bubbles can flow out during priming, wherein an inner peripheral surface of the prime port includes a recessed portion recessed radially outward, and a continuous portion gently formed continuously to the recessed portion, and
a straight line passing from the proximal end point of the blood outflow port through the center point of the housing is inclined with respect to the virtual center line of the housing at least to a site exceeding the recessed portion in a circumferential direction.

3. The oxygenator according to claim 1 or 2, wherein an intersection angle of the straight line passing from the proximal end point of the blood outflow port through the center point of the housing with respect to the virtual center line of the housing is larger than 0 degrees and 16 degrees or shorter.

4. An oxygenator comprising:
a cylindrical housing;
a cylindrical core stored in the housing;
a hollow fiber membrane layer formed by a bundle of a hollow fiber membrane wound around the core;
a first blood chamber formed between an outer peripheral surface of the core and an inner peripheral surface of the hollow fiber membrane layer;
a second blood chamber formed between an outer peripheral surface of the hollow fiber membrane layer and an inner peripheral surface of the housing;
a blood inflow port that is provided on the core so as to extend in a longitudinal direction of the core and communicates with the first blood chamber; and
a blood outflow port that is provided on the housing so as to extend in a direction intersecting with a longitudinal direction of the housing and in a tangential direction of the housing and communicates with the second blood chamber, wherein, in a side view, a proximal end point of the blood outflow port is offset from a virtual center line of the housing in the tangential direction of the housing.

5. An oxygenator comprising:
a cylindrical housing;
a cylindrical core stored in the housing;
a hollow fiber membrane layer formed by a bundle of a hollow fiber membrane wound around the core;
a first blood chamber formed between an outer peripheral surface of the core and an inner peripheral surface of the hollow fiber membrane layer;
a second blood chamber formed between an outer peripheral surface of the hollow fiber membrane layer and an inner peripheral surface of the housing;
a blood inflow port that is provided on the core so as to extend in a longitudinal direction of the core and communicates with the first blood chamber; and
a blood outflow port that is provided on the housing so as to extend in a direction intersecting with a longitudinal direction of the housing and in a tangential direction of the housing and communicates with the second blood chamber, wherein, in a side view, a projection projecting radially inward from an inner peripheral surface of the housing is provided in a region where a straight line passing from a proximal end point of the blood outflow port through a center point of the housing intersects with the inner peripheral surface of the housing, on a side opposite to the proximal end point.

6. The oxygenator according to claim 5, wherein the projection is formed in a mountain shape so that an amount projecting outward from the center decreases.

7. The oxygenator according to claim 5 or 6, wherein a projection amount of a most projecting site of the projection is larger than 0% and smaller than 5% of an inner diameter of the housing.
